# EUROPEAN PATENT SPECIFICATION

(11) **EP 4 081 165 B1**
(45) Date of publication and mention of the grant of the patent: **13.05.2026**
(21) Application number: 20907128.1
(22) Date of filing: 24.12.2020
(51) Int. Cl.: A61F 2/16, A61L 31/10, A61L 31/14

(54) **BIOCOMPATIBLE NATURAL POLYMER BASED CARTRIDGE COATING FOR INTRAOCULAR LENS (IOL) IMPLANTATION AND A PRODUCTION METHOD THEREOF**
BIOKOMPATIBLE PATRONENBESCHICHTUNG BASIEREND AUF NATÜRLICHEM POLYMER FÜR DIE IMPLANTATION VON INTRAOKULAREN LINSEN (IOL) UND EIN PRODUKTIONSVERFAHREN DAFÜR
REVÊTEMENT DE CARTOUCHE À BASE DE POLYMÈRE NATUREL BIOCOMPATIBLE POUR L'IMPLANTATION DE LENTILLE INTRAOCULAIRE (LIO) ET SON PROCÉDÉ DE PRODUCTION

(30) Priority: 24.12.2019 TR 201921360
(43) Date of publication of application: 02.11.2022
(73) Proprietor: VSY Biyoteknoloji Ve Ilac Sanayi Anonim Sirketi, Istanbul (TR)
(72) Inventor: OYTUN, Faruk, Tuzla/Istanbul (TR); ATMACA, Serkan, Tuzla/Istanbul (TR)
(74) Representative: Sevinç, Cenk
(86) International application number: PCT/TR2020/051370
(87) International publication number: WO 2021/133349

(56) References cited:
- WO-A1-2011/057131
- CN-A- 1 883 721
- JP-A- 2007 021 010
- US-A1- 2003 134 132
- US-A1- 2005 147 735
- US-A1- 2005 256 528
- US-A1- 2015 182 673
- US-B2- 8 821 572

## Description

### Field of the Invention

The present invention relates to biocompatible natural polymer-based coatings which are used as the inner coating material for the cartridge of intraocular lenses (IOL) and facilitate the implantation of lenses.

### Background of the Invention

The natural crystal lens in the human eye loses its transparency over time due to old age, impact or some diseases, and the vision deteriorates with the decrease of light reaching the retina. In this case, intraocular lenses (IOL) are implanted into the eye in place of the natural crystal lens after cataract surgery. An incision is made in the eye during the implantation of intraocular lenses (IOL). This incision should be as small as possible to reduce trauma and accelerate recovery.

In the state of the art, these intraocular lenses (IOL) were made of polymethyl methacrylate (PMMA) material in the early days due to its biocompatible feature. Since PMMA is a hard polymer, a 5-7 mm incision was required for its implantation. Since an incision of this size requires suture, it decreases the comfort of the patient and prolongs the healing process. Implantation performed with smaller incision sizes not only eliminates the need for sutures, but also accelerates the healing process of the patient. For this reason, today, acrylic based foldable, flexible intraocular lenses (IOLs) having both hydrophilic and hydrophobic properties are produced. These lenses can be implanted into the eye even in incision sizes of 3 mm or less.

Cartridge injector systems are used in cataract surgeries for implanting the lens into the eye. The lens is folded in the cartridge and passed through the small diameter cartridge tunnel, and then unfolded in the lens capsule located in the eye.

IOL cartridges are generally produced from polymers such as polyolefin (e.g. polypropylene) having high hydrophobic properties. When the IOL is pushed inside the cartridge made of these polymers having high friction force, the movement of the lens inside the cartridge is prevented. As the pressure increases, IOL which is folded inside the cartridge has a tendency to expand inside the cartridge with the effect of the friction force, and it becomes impossible to come out of the end of the cartridge. In this case, implantation fails and the IOL undergoes physical deformations such as rupture, tear, and scratching.

Recently, different methods have been used to minimize the friction in the cartridge and facilitate the implantation of the intraocular lens (IOL) by coming out of the cartridge tip. One of these methods is adding fatty acid esters such as glycerol monostearate (GMS) to the material of the cartridge as a lubricant additive in the production process. The cartridges produced with this method are subjected to high temperatures for the lubricant additive to impregnate into the inner surface of the cartridge. Even though the cartridge to which a lubricant is added provides a very effective slippery coating, these fatty acid esters (lubricant) rise to the surface of the cartridge over time. This lubricant material, which has risen to the surface, may adhere to the surface of the intraocular lens (IOL) during its long shelf life, causing its optical properties to be damaged. Therefore, the shelf life of the cartridges produced by this method should be kept short.

Generally during the application, a viscoelastic gel is added between the lens surface and the coated cartridge surface, allowing the lens and the cartridge to be activated and the lens to slide easily inside the cartridge. For example, important risks and disadvantages such as inability to adjust the viscoelastic fluid used for sliding the lens during surgeries, inability to spread viscoelastic gel homogeneously, strains resulting during the delivery of the lens and scratches and deformations occurring in the lens when low amount of viscoelastic gel is used, as well as difficulty in cleaning after the implantation, the risk of changing the optical properties of the lens by covering the surface of the lens when higher amount of viscoelastic gel is used can be experienced.

United States patent document no US8323799B2, an application known in the state of the art, discloses that a solution formed by formulating polyvinyl prolidone (PVP) or hyaluronic acid (HA) as a hydrophilic lubricant, a commercial urethane dispersion (NeoRez^{®} R-9330) as a matrix polymer and polyfunctional aziridine as a crosslinking agent is used as an IOL cartridge coating material. Within the scope of the said application, it is stated that the cartridges subjected to plasma treatment are coated with the prepared coating solution and then left to dry overnight at 60°C.

Another method known in the art is to apply a polymer-based lubricant film coating on the inner surface of the cartridge. In patent applications US6238799B1 and US6866936B2 made in accordance with this method, mixtures comprising polyacrylates, polymethacrylates, polyurethanes, polyethylene and polypropylene copolymers, polyvinyl chlorides, epoxides, polyamides, polyesters and alkyd copolymers as matrix polymer; poly(N-vinyl lactams), poly(vinylpyrrolidone), poly(ethylene oxide) polypropylene oxide) polyacrylamides, cellulosics, methyl cellulose, polyacrylic acids, polyvinyl alcohols, and polyvinyl ethers as hydrophilic polymers; and at least one crosslink agent are used as IOL coating material. The coating process is performed by applying this mixture to IOL cartridges. The lubricant coatings disclosed in the said applications are relatively hard and non-flexible. This situation has a risk that the cartridge inside the cartridge may detach from the coated surface due to its hard structure and may damage the lens during the implantation process.

United States patent document no US8821572B2, an application known in the state of the art, discloses that IOL coating material comprises polyurethane and PVP which is a hydrophilic polymer and a cross-linking agent. Here, it is aimed to apply the coating directly to the inner surface of the cartridge as a single layer.

An article titled *"Preparation and evaluation of a lubricious treated cartridge used for* implantation *of intraocular lenses",* one of the applications known in the state of the art, discloses that cartridges subjected to plasma treatment are immersed in a coating solution formed by using polyethylene imine (PEI), PVP as IOL coating material and glutaraldehyde as crosslinking agent and cured at °70 C. It is explained that after the lenses are placed in the coated cartridges, viscoelastic gel is injected before implantation to help the lens slide more easily, and it is waited for 4.5 minutes the lens to be activated. This is quite a long time and carries great risks during the surgical operation. Implantation should be performed in a short time after both viscoelastic gel and saline solution, which will ensure that the lens and coating material are activated, are added.

United States patent document no US20170128195A1, an application known in the state of the art, discloses a solution comprising polyurethane and fluorescing sodium salt as IOL coating material and polyfunctional aziridine which is a crosslinking agent. It is stated that the coated cartridges are exposed to UV light of 254 nm and the indicator properties are observed whether the fluorescent salt showing fluorescent properties is coated homogeneously on the cartridge.

JP2007/021010A relates to a coating composition containing a zwitterionic compound and a hydrophilic polymer, which is crosslinked to improve lubricity on medical devices such as intraocular lens (IOL) inserters. However, the approach described in JP2007/021010A lacks the use of natural, biodegradable polymers with complementary functional groups (e.g., amine and carboxylic acid groups) that react to form a crosslinked network without synthetic solvents.

US2005/256528A1 concerns an IOL insertion device with a bilaminar lubricious coating where a top coat, typically hyaluronan, is grafted onto a base acrylic polymer layer. While it aims to improve lubricity and permanence, US2005/256528A1 does not utilize biodegradable, natural polymers or a twolayer system formed by electrostatic interactions between amine- and carboxylfunctionalized biopolymers.

### Summary of the Invention

The objective of the invention is to provide a biodegradable and biocompatible coating material for an intraocular lens cartridge comprising completely natural polymer.

Another objective of the invention is to obtain a coating material that shows successful results in the delivery of both hydrophobic and hydrophilic lenses.

Another objective of the invention is to provide a water-based coating material which carries neither stability nor health risks which are seen in other solventbased formulations used in the literature.

Yet another objective of the present invention is to provide a coating material which can easily be activated even with saline solution that is widely available in surgical environments instead of viscoelastic gel in conventional applications, and therefore which enables to save time during surgery and reduce costs.

### Detailed Description of the Invention

**"Biocompatible Natural Polymer Based Cartridge Coating for Intraocular Lens Implantation and A Production Method Thereof"** developed to fulfil the objectives of the present invention is illustrated in the accompanying figure, wherein:

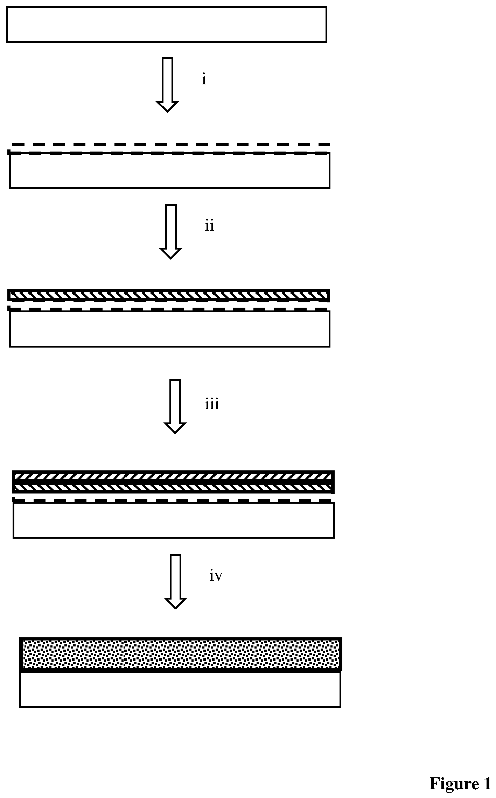

**Figure 1** is schematic view of the method for applying coating on the surface of intraocular lens (IOL) cartridge within the scope of the invention.

The coating for the intraocular lens (IOL) cartridge of the present invention according to claim 1 is used as a coating on the inner surface of the intraocular lens cartridges, and it is a biocompatible natural polymer-based material which facilitates the implantation of intraocular lenses. The subject matter of the invention, in relation to the prior art, enables to develop a coating which will enable the intraocular lens (IOL) to be easily implanted through the cartridge without damaging it, remains stable during its long shelf life, and is flexible and lubricious.

Within the scope of the invention, two types of biocompatible and biodegradable natural polymers are used in terms of functional properties. As the first type, amine functionalized natural polymers **(chitosan (CHI), O-carboxymethyl chitosan (O-CM-CHI),)** are used; and as the second type, carboxylic acid functionalized natural polymers **(hyaluronic acid (HA), carboxymethyl cellulose (CMC), N,O-carboxymethyl chitosan (N, O-CM-CHI))** are used. 1-**Ethyl-3- (3-dimethylaminopropyl) carbodiimide** (EDC) is used as a crosslinking agent in order to crosslink the polymers with each other.

The coating method provided within the scope of the invention according to claim 6 is comprised of the following steps:
i. Applying plasma treatment to the cartridges of the intraocular lens (IOL),
ii. Coating amine functionalized polymer on the surface of the cartridge,
iii. Adding the crosslinking agent to the carboxylic acid functionalized polymer,
iv. Then, coating carboxylic acid functionalized polymer on the amine functionalized polymer coating on the surface of the cartridge,
v. Applying curing process on the coating surfaces.

### Coating Process

The coating process is applied to the inner surface of plasma treated polypropylene (PP) cartridges of formulations of different compositions. Centrifugal coating method is preferably used as the coating method within the scope of the invention. With this method, a homogeneous film can be obtained by removing the excess polymer solution remaining on the surface with the effect of centrifugal force. The application is carried out based on the values in Table 1 as coating parameters.

**Table 1**

| **Parameters** | **Value** |
|---|---|
| Volume of the coating solution (µL)* | 30-100 |
| Centrifuge rotation speed (rpm) | 250-1750 |
| Centrifuge time (sec) | 5-40 |

| | |
|---|---|
| * These are the volumes for both (amine functionalized and carboxylic functionalized) polymer solutions separately. | |

The formulations, the ratios of which by weight are indicated in Table 2, are prepared for coating studies. The crosslinking agent is added to the carboxylic acid functionalized polymer solutions before coating. Chitosan (CHI) solutions are prepared in 0.5% acetic acid and all other polymer solutions are prepared with deionized water.

**Table 2**

| | **CM-001** | **CM-002** | **CM-003** | **CM-004** | **CM-005** |
|---|---|---|---|---|---|
| **Amine functionalized polymer (by weight %)** | 0.1- 0.8% CHI | 0.1- 0.8% CHI | 0.1- 0.8% CHI | 0.1- 0.8% CHI | 0.1- 0.8% CHI |
| **Carboxylic acid functionalized polymer (by weight %)** | 0.1- 0.8% HA | 0.1- 0.8% CMC | 0.1- 0.8% HA | 0.1- 0.8% CMC | 0.050- 0.60% HA |
| | | | | | % 0.050- 0.60 CMC |
| **Crosslinking agent (by weight %)** | - | - | 0.050- 0.25% EDC | 0.050- 0.25% EDC | 0.050- 0.25% EDC |

The coating process carried out within the scope of the invention goes through the following stages;
1. The cartridge surface is made suitable for coating by reducing the surface energies of the polypropylene (PP) cartridges by means of subjecting them to plasma treatment.
2. Amine functionalized polymer solution is dropped on the cartridges placed on the centrifuging device.
3. The centrifuge process is started and thereby the polymer solution is enabled to be distributed homogeneously inside the cartridge, and the excess solution is removed.
4. Carboxylic acid functionalized polymer solution is dropped onto the cartridges coated with amine functionalized polymer.
5. The centrifuge process is started and thereby the polymer solution is enabled to be distributed homogeneously inside the cartridge, and the excess solution is removed.
6. The obtained cartridges are cured at a temperature of 50-80 °C for 1-4 hours.

Within the scope of the experimental studies carried out while developing the invention, the delivery tests of acrylic based hydrophobic intraocular lenses (IOL) with the cartridges obtained as a result of the curing process were performed. The evaluation of the delivery tests was carried out using the same type of intraocular lenses (IOL (Hydrophobic Acrylic IOL, 29D)) with the same diopter power. The delivery tests have been carried out with the following process steps:
1. Placing the lens in the coated cartridge
2. Mounting the cartridge into the injector
3. The saline solution penetrating into the areas on the inner part of the lens and cartridge
4. Carrying out delivery tests under optic microscope

Table 3 shows the results of the delivery tests:

**Table 3**

| | **Hydrophobic IOL** | **Injection Force (N)** |
|---|---|---|
| CM-001 | + | - |
| CM-002 | + | - |
| CM-003 | +++ | 7.8 N |
| CM-004 | +++ | 10.4 N |
| CM-005 | +++ | 8.9 N |
| +++ Passed | ++ Passed with difficulty | + Did not pass |

As it can also be seen from the delivery tests, lens deliveries were unsuccessful in formulations without crosslinking agents (CM-001 and CM-002) (Table 3). Since polymer coatings that are not crosslinked cannot be activated sufficiently with the saline solution, they cannot exhibit a good lubrication between the lens and the cartridge. In formulations comprising crosslinking agents, it is seen that the delivery has been successful. This shows that by taking the saline solution therein, the cross-linked mesh structure, forms an active layer between the lens and the cartridge. The crosslinking agent enables the carboxylic acid group in the HA and/or CMC polymers to react with the amine group in the CHI polymer and to be linked to each other. It acts as a catalyst during the reaction, and establishes the crosslink structure, and then it is removed from the coating by the washing process. Furthermore, in formulations where crosslinking agents are used, the film layer inside the cartridge establishes a good bond with the cartridge surface and does not leave any residue on the lens during delivery. The cross-linked film layer, which has a soft and flexible structure, does not damage the lens during implantation.

Table 3 also shows the injection forces applied to the injector during the delivery in Newtons (N). Injection force tests of the samples, the delivery tests of which have not been carried out, are excluded from the scope of the products developed within the scope of the invention. Referring to Table 3, the force values applied to the samples that have successfully passed the delivery test are very low. These low values mean that the surgeon can carry out the implantation of the lens into the eye more easily during the surgical operation. In systems with high injection force, the lens can be damaged and this poses a great risk during the surgery.

The advantages of the coating material obtained within the scope of the invention before the state of the art can be listed as follows:
➢ The said coating material obtained within the scope of the invention is completely natural polymer based and has biocompatible properties.
➢ The polymers used in the formulation also exhibit biodegradable properties and do not show adverse effects in the body.
➢ The HA present in the formulation is a very important polymer produced by the human body, and it has high water holding capacity due to its very high hydrophilic properties. This feature enables the HA to show significant lubricant properties on the cartridge as coating material.
➢ The formulation shows successful results in delivery of both hydrophobic and hydrophilic lenses.
➢ The coating material obtained within the scope of the invention is water based, and it does not have the stability and health risks which are seen in other solvent based formulations used in the literature.
➢ Viscoelastic gel is injected inside the cartridge in order to activate the lens and cartridge coating during implantation of the lens to facilitate sliding of the lens. The coating material of the invention can easily activate the lens cartridge even with saline solution that is commonly found in surgical environments, and it can facilitate the delivery of the lens.
➢ Being able to use saline solution instead of viscoelastic gel enables to save time during surgery as well as it also reduces the costs.
➢ Being able to use saline solution instead of viscoelastic gel eliminates the disadvantages of viscoelastic gel's inability to enter between the lens and the cartridge especially in preloaded systems and in this case not being able to enable the delivery of the lens. The saline solution easily penetrates into the narrowest areas and becomes activated with the natural polymer coating material provided on the lens and cartridge surface and allows the delivery.

## Claims

1. Coating for intraocular lens (IOL) cartridge; which is developed in order to facilitate implantation of intraocular lenses (IOL), to enable the implantation of intraocular lens (IOL) through the cartridge easily without damaging it, to enable it to be stable during its long shelf life, to be flexible and lubricious; comprising
- At least one amine functionalized natural polymer which is selected from a group comprised of chitosan (CHI), O-carboxymethyl chitosan (O-CM-CHI), and mixtures thereof,
- At least one carboxylic acid functionalized natural polymer which is selected from a group comprised of hyaluronic acid (HA), carboxymethyl cellulose (CMC), N,O-carboxymethyl (N,O-CM-CHI) chitosan and mixtures thereof,
- At least one crosslinking agent

2. Coating for intraocular lens (IOL) cartridge according to claim 1, wherein polymer solutions are prepared in deionized water solvent.

3. Coating for intraocular lens (IOL) cartridge according to claim 1, wherein chitosan (CHI) solutions are prepared in 0.5 % acetic acid.

4. Coating for intraocular lens (IOL) cartridge according to claim 1, comprising 1-Ethyl-3-(3-dimethylaminopropyl) carbodiimide (EDC) as crosslinking agent.

5. Coating for intraocular lens (IOL) cartridge according to any one of the preceding claims, comprising 0.1-0.8 % by weight of chitosan (CHI), 0-0.8 % by weight of carboxymethyl cellulose (CMC), 0-0.8 % by weight of hyaluronic acid (HA), and 0-0.25 % by weight of 1-ethyl-3-(3-dimethylaminopropyl) carbodiimide (EDC).

6. Production method of coating for intraocular lens (IOL) cartridge according to any one of the preceding claims, comprising the steps of:
i. Applying plasma treatment to intraocular lens (IOL) cartridges for 10-50 seconds at power of 30-90 W,
ii. Coating amine functionalized polymer on the surface of the cartridge,
iii. Adding the crosslinking agent to the carboxylic acid functionalized polymer,
iv. Then, coating carboxylic acid functionalized polymer on the amine functionalized polymer coating on the surface of the cartridge,
v. Applying curing process on the coating surfaces.

7. Production method of coating for intraocular lens (IOL) cartridge according to claim 6, wherein the polymer is applied on the cartridge surface by dropping in the process steps of coating amine functionalized polymer and coating carboxylic acid functionalized polymer on the cartridge surface.

8. Production method of coating for intraocular lens (IOL) cartridge according to claim 6, wherein the cartridge taken to the centrifuge device is centrifuged to homogeneously distribute the polymer solution and to remove excess solution following the process steps of coating amine functionalized polymer and coating carboxylic acid functionalized polymer on the cartridge surface.

9. Production method of coating for intraocular lens (IOL) cartridge according to claim 8, wherein centrifuge rotation speed is 250-1750 rpm and its time is 5-40 seconds.

10. Production method of coating for intraocular lens (IOL) cartridge according to claim 6, wherein curing process is carried out on the coating surfaces at a temperature of 50-80°C for 1-4 hours.

## Patentansprüche

1. Beschichtung für Intraokularlinsen (IOL)-Patrone, die entwickelt wurde, um die Implantation von Intraokularlinsen (IOL) zu erleichtern, um die Implantation von Intraokularlinsen (IOL) durch die Patrone leicht zu ermöglichen, ohne sie zu beschädigen, um zu ermöglichen, dass sie während ihrer langen Haltbarkeit stabil ist, um flexibel und gleitfähig zu sein; umfassend
- Mindestens ein aminfunktionalisiertes natürliches Polymer, das aus einer Gruppe ausgewählt ist, die aus Chitosan (CHI), O-Carboxymethylchitosan (O-CM-CHI) und Mischungen davon besteht,
- Mindestens ein mit Carbonsäure funktionalisiertes natürliches Polymer, das aus einer Gruppe ausgewählt ist, die Hyaluronsäure (HA), Carboxymethylcellulose (CMC), N,O-Carboxymethyl (N,O-CM-CHI) Chitosan und Mischungen davon umfasst,
- Mindestens ein Vernetzungsmittel

2. Beschichtung für Intraokularlinsen (IOL)-Patrone nach Anspruch 1, wobei die Polymerlösungen in deionisiertem Wasser als Lösungsmittel hergestellt werden.

3. Beschichtung für Intraokularlinsen(IOL)-Patrone nach Anspruch 1, wobei Chitosan(CHI)-Lösungen in 0,5 %iger Essigsäure hergestellt werden.

4. Beschichtung für Intraokularlinsen (IOL)-Patrone nach Anspruch 1, umfassend 1-Ethyl-3-(3-dimethylaminopropyl)-carbodiimid (EDC) als Vernetzungsmittel.

5. Beschichtung für Intraokularlinsen (IOL)-Patrone nach einem der vorhergehenden Ansprüche, umfassend 0,1-0,8 Gew.-% Chitosan (CHI), 0-0,8 Gew.-% Carboxymethylcellulose (CMC), 0-0,8 Gew.-% Hyaluronsäure (HA) und 0-0,25 Gew.-% 1-Ethyl-3-(3-dimethylaminopropyl)-carbodiimid (EDC).

6. Verfahren zur Herstellung einer Beschichtung für eine Intraokularlinsen (IOL)-Patrone nach einem der vorhergehenden Ansprüche, umfassend die Schritte:
i. Plasmabehandlung von Intraokularlinsen (IOL)-Kartuschen für 10-50 Sekunden bei einer Leistung von 30-90 W,
ii. Beschichtung der Oberfläche der Kartusche mit einem aminfunktionalisierten Polymer,
iii. Hinzufügen des Vernetzungsmittels zu dem mit Carbonsäure funktionalisierten Polymer,
iv. Anschließend Beschichtung des mit Carbonsäure funktionalisierten Polymers auf der mit Amin funktionalisierten Polymerbeschichtung auf der Oberfläche der Kartusche,
v. Anwendung des Aushärtungsprozesses auf die Beschichtungsoberflächen.

7. Verfahren zur Herstellung einer Beschichtung für eine Intraokularlinsen-(IOL)-Patrone nach Anspruch 6, wobei das Polymer auf die Patronenoberfläche aufgetragen wird, indem es in den Verfahrensschritten des Auftragens von aminfunktionalisiertem Polymer und des Auftragens von carbonsäurefunktionalisiertem Polymer auf die Patronenoberfläche getropft wird.

8. Verfahren zur Herstellung einer Beschichtung für eine Intraokularlinsen (IOL)-Kartusche nach Anspruch 6, wobei die in die Zentrifugenvorrichtung eingebrachte Kartusche zentrifugiert wird, um die Polymerlösung homogen zu verteilen und überschüssige Lösung nach den Verfahrensschritten der Beschichtung mit aminfunktionalisiertem Polymer und der Beschichtung mit carbonsäurefunktionalisiertem Polymer auf der Kartuschenoberfläche zu entfernen.

9. Verfahren zur Herstellung einer Beschichtung für eine Intraokularlinsen-(IOL)-Patrone nach Anspruch 8, wobei die Rotationsgeschwindigkeit der Zentrifuge 250-1750 U/min und ihre Dauer 5-40 Sekunden beträgt.

10. Verfahren zur Herstellung einer Beschichtung für eine Intraokularlinsen (IOL)-Patrone nach Anspruch 6, wobei der Härtungsprozess an den Beschichtungsoberflächen bei einer Temperatur von 50-80°C für 1-4 Stunden durchgeführt wird.

## Revendications

1. Revêtement pour cartouche de lentille intraoculaire (LIO) ; développé afin de faciliter l'implantation de lentilles intraoculaires (LIO), de permettre l'implantation de lentilles intraoculaires (LIO) à travers la cartouche facilement sans l'endommager, de lui permettre d'être stable pendant sa longue durée de conservation, d'être flexible et lubrifiant ; comprenant
- Au moins un polymère naturel fonctionnalisé par une amine, choisi dans le groupe constitué par le chitosane (CHI), le O-carboxyméthylchitosane (O-CM-CHI) et leurs mélanges,
- Au moins un polymère naturel fonctionnalisé par un acide carboxylique choisi dans le groupe comprenant l'acide hyaluronique (HA), la carboxyméthylcellulose (CMC), le N,O-carboxyméthyl (N,O-CM-CHI) chitosane et leurs mélanges,
- Au moins un agent de réticulation

2. Revêtement pour cartouche de lentille intraoculaire (LIO) selon la revendication 1, dans lequel les solutions polymères sont préparées dans un solvant à base d'eau déionisée.

3. Revêtement pour cartouche de lentille intraoculaire (LIO) selon la revendication 1, dans lequel les solutions de chitosane (CHI) sont préparées dans de l'acide acétique à 0,5 %.

4. Revêtement pour cartouche de lentille intraoculaire (LIO) selon la revendication 1, comprenant du 1-Éthyl-3-(3-diméthylaminopropyl)carbodiimide (EDC) comme agent de réticulation.

5. Revêtement pour cartouche de lentille intraoculaire (IOL) selon l'une quelconque des revendications précédentes, comprenant 0,1 à 0,8 % en poids de chitosane (CHI), 0 à 0,8 % en poids de carboxyméthylcellulose (CMC), 0 à 0,8 % en poids d'acide hyaluronique (HA) et 0 à 0,25 % en poids de 1-éthyl-3-(3-diméthylaminopropyl)carbodiimide (EDC).

6. Procédé de fabrication d'un revêtement pour cartouche de lentille intraoculaire (LIO) selon l'une quelconque des revendications précédentes, comprenant les étapes suivantes :
i. Application d'un traitement au plasma sur les cartouches de lentilles intraoculaires (LIO) pendant 10 à 50 secondes à une puissance de 30 à 90 W,
ii. Revêtement de la surface de la cartouche par un polymère fonctionnalisé à l'amine,
iii. Ajout de l'agent de réticulation au polymère fonctionnalisé à l'acide carboxylique,
iv. Puis, revêtement du polymère fonctionnalisé à l'acide carboxylique sur le revêtement de polymère fonctionnalisé à l'amine sur la surface de la cartouche,
v. Application de processus de durcissement sur les surfaces de revêtement.

7. Procédé de fabrication d'un revêtement pour cartouche de lentille intraoculaire (LIO) selon la revendication 6, dans lequel le polymère est appliqué sur la surface de la cartouche par gouttelettes au cours des étapes de revêtement de la cartouche avec un polymère à fonction amine et d'un polymère à fonction acide carboxylique.

8. Procédé de fabrication d'un revêtement pour cartouche de lentille intraoculaire (LIO) selon la revendication 6, dans lequel la cartouche amenée au dispositif centrifuge est centrifugée afin de répartir de manière homogène la solution polymère et d'éliminer l'excès de solution après les étapes de revêtement de la surface de la cartouche avec un polymère fonctionnalisé par une amine et un polymère fonctionnalisé par un acide carboxylique.

9. Procédé de fabrication d'un revêtement pour cartouche de lentille intraoculaire (LIO) selon la revendication 8, dans lequel la vitesse de rotation de la centrifugeuse est comprise entre 250 et 1750 tr/min et sa durée est comprise entre 5 et 40 secondes.

10. Procédé de fabrication d'un revêtement pour cartouche de lentille intraoculaire (LIO) selon la revendication 6, dans lequel le processus de durcissement est effectué sur les surfaces de revêtement à une température de 50 à 80 °C pendant 1 à 4 heures.
